# EUROPÄISCHE PATENTSCHRIFT

(11) **EP 0 539 805 B1**
(45) Veröffentlichungstag und Bekanntmachung des Hinweises auf die Patenterteilung: **03.09.1997**
(21) Anmeldenummer: 92117672.3
(22) Anmeldetag: 16.10.1992
(51) Int. Cl.: C07D 237/30, C07D 237/32, C07D 401/12, C07D 453/02, A61K 31/50

(54) **Neue Phthalazine, die in 1-Stellung eine Ether-Gruppierung enthalten und Verfahren zu deren Herstellung**
Phthalazines substituted on position 1 by an ether group and process for their preparation
Phthalazines substituées en position 1 par un groupe éther et procédé pour leur préparation

(30) Priorität: 31.10.1991 DE 4135910
(43) Veröffentlichungstag der Anmeldung: 05.05.1993
(73) Patentinhaber: ASTA Medica Aktiengesellschaft, D-01277 Dresden (DE)
(72) Erfinder: Scheffler, Gerhard, Dr., W-6454 Bruchköbel (DE); Fleischhauer, Ilona, Dr., W-6050 Offenbach (DE); Kutscher, Bernhard, Dr., W-6457 Maintal 1 (DE); Engel, Jürgen, Prof., W-8755 Alzenau (DE); Szelenyi, Stefan, Prof., W-8501 Schwaig (DE); Werner, Ulrich, Dr., W-5429 Miehlen (DE)
(74) Vertreter: Decker, Karl-Ludwig

(56) Entgegenhaltungen:
- FR-A- 2 288 518
- CHEMICAL ABSTRACTS, vol. 51, no. 22, 25. November 1957, Columbus, Ohio, US; abstract no. 17927i, ISAO SATODA ET AL.
- CHEMICAL ABSTRACTS, vol. 58, no. 4, 18. Februar 1963, Columbus, Ohio, US; abstract no. 3425d, EISAKU HAYASHI ET AL.
- CHEMICAL ABSTRACTS, vol. 84, no. 9, 1. M rz 1976, Columbus, Ohio, US; abstract no. 58116t, K. GRABLIAUSKAS ET AL.
- CHEMICAL ABSTRACTS, vol. 88, no. 19, 8. Mai 1978, Columbus, Ohio, US; abstract no. 136646q,
- PATENT ABSTRACTS OF JAPAN vol. 5, no. 160 (C-75)(832) 15. Oktober 1981 & JP-A-5690066
- PATENT ABSTRACTS OF JAPAN vol. 12, no. 70 (C-479)(2917) 4. M rz 1988

## Beschreibung

In J. Prakt. Chemie 2, 51 (1895) Seite 148 u. 149 wird die Herstellung der Verbindungen 1-Methoxyphthalazin sowie 1 Ethoxy-phthalazin aus 1-Chlorphthalazin und Natriummethylat beziehungsweise Natriumethylat beschrieben. In dem US-Patent 2 484 029 wird 1-Phenoxy-phthalazin als Ausgangssubstanz für die Herstellung von 1 Hydrazino-phthalazin genannt, ebenso in CA, vol. 51, No. 22, abstract No. 17927i.

Die Herstellung von 1-Propoxy-phthalazin wird in CA, vol. 58, No. 4, abstract No. 3425d aufgezeigt.

In CA, vol. 84, No. 9,abstract No. 58116t werden 1(2H)-phthalazthione, die in 1-Stellung unsubstituiert sind, beschrieben. In JP-A-56-90066 werden Phenylacetonitrile beschrieben, die mit phthalazin-1-on-2-yl substituiert sein können. Diese Nitrile stellen Zwischenprodukte für Verbindungen mit antiinflammatorischen und analgetischen Eigenschaften dar. In FR-A-2288 518 werden araliphati-sche heterocyclische Verbindungen beansprucht, die mit 3,4-dihydro-4-oxo-phthalazin-1-yl-amino substituiert sein können.

Der Erfindung liegt also die Aufgabe zugrunde, weitere Verbindungen zur Verfügung zu stellen, die analgetische, antiphlogistische, antikonvulsive und antipyretische Wirkungen besitzen.

Die Erfindung betrifft die durch die Patentansprüche definierten Gegenstände, wobei die erfindungsgemäßen Verbindungen der allgemeinen Formel I wie folgt definiert sind: 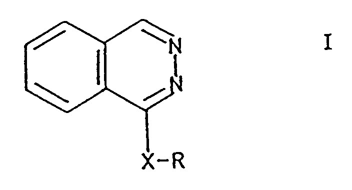 worin X Sauerstoff bedeutet und der Rest R einen Chinuclidylrest,einen Phenylrest, einen Pyridylrest, einen durch die Reste R₁, R₂ und/oder R₃ substituierten Phenyl- oder Pyridylrest, einen durch Pyridyl oder Alkylpyridyl substituierten C₁-C₆-Alkylrest oder einen durch Phenyl substituierten C₁-C₆-Alkylrest darstellt, wobei jener Phenylrest auch durch die Reste R₁, R₂ und/oder R₃ substituiert sein kann und die Reste R₁, R₂ und R₃ gleich oder verschieden sind und Wasserstoff, Halogen, Trihalogenmethyl, C₁-C₆-Alkyl,C₁-C₆-Alkoxy, Carboxy,Carb-C₁-C₆-alkoxy, Amino, C₁-C₆-Alkylamino, C₁-C₆-Dialkylamino, C₁-C₆-Trialkylamino, C₂-C₆-Alkanoylamino oder C₂-C₆-Alkanoylamino, welches im Alkylteil noch eine Aminogruppe enthält, bedeuten und deren physiologisch verträgliche Säureadditionssalze, ausgenommen 1-Phenoxy-phthalazin

Die erfindungsgemäßen Verbindungen sind pharmakologisch wirksam. Insbesondere besitzen die erfinungsgemäßen Verbindungen eine ausgeprägte und stark analgetische (zum Beispiel peripher analgetisch,zentralanalgetisch), antiphlogistische, antikonvulsive und antipyretische Wirkung.

Die folgenden Erläuterungen stellen wichtige beispielhafte Angaben dar:

Die in der Formel 1 vorkommenden Alkoxygruppen, Alkanoylaminogruppen oder Alkanoylgruppen können gerade oder verzweigt sein. Dasselbe gilt auch für Alkyl- und Alkoxygruppen, falls diese Bestandteil anderer zusammengesetzter Gruppen sind (zum Beispiel in Form einer Monoalkyl-, Dialkyl- oder Trialkylamino-gruppe, Alkanoylaminogruppe oder Carbalkoxygruppe).
Die Alkyl- und Alkoxygruppen als solche oder als Bestandteil von anderen zusammengesetzten Gruppen bestehen insbesondere aus 1-4 C-Atomen, vorzugsweise 1 oder 2 C-Atomen. Alkanoylgruppen oder Alkanoylamino-gruppen bestehen insbesondere aus 2-4 vorzugsweise 2-3 C-Atomen. Alkylpyridyl bedeutet einen Pyridylrest, der eine, zwei oder auch drei C₁-C₆-Alkylreste, vorzugsweise den Methylrest, enthält. Die Alkylreste befinden sich vorzugsweise in 2-,3-,4- und/oder 6-Stellung des Pyridylrestes.

Der Pyridylrest selbst ist vorzugsweise über die 2-, 4-und/oder 6-Stellung mit dem C₁-C₆-Alkylrest verbunden. Dies gilt auch entsprechend, wenn das Symbol R einen Pyridylrest bedeutet.
Vorzugsweise handelt es sich bei dem C₁-C₆--Alkylrest, welcher durch Pyridyl- oder Alkylpyridyl substituiert ist um einen Pyridylmethylrest das heißt einen Methylrest, welcher vorzugsweise einen Pyridyl-(2)-, Pyridyl-(4)- oder Pyridyl-(6)-rest enthält, wobei der Pyridinring gegebenenfalls auch zusätzlich eine, zwei- oder drei-C₁-C₆-Alkylreste, vorzugsweise Methylreste enthalten kann.

Als Chinuclidinring kommt vorzugsweise der Chinuclidyl-(3)-rest in Frage.

X bedeutet Sauerstoff.
Besonders wirksam sind solche Verbindungen der Formel I, worin X Sauerstoff ist und R Phenyl oder Phenyl, welches eine Aminogruppe oder eine C₂-C₄-Alkanoylaminogruppe, vorzugsweise in der 4-Stellung, enthält.

Je nach den Verfahrensbedingungen und Ausgangsstoffen erhält man die Endstoffe der Formel 1 in freier Form oder in Form ihrer Salze. Die Salze der Endstoffe (falls diese ein basisches Stickstoffatom enthalten) können in an sich bekanner Weise beispielsweise mit Alkali oder Ionenaustauschern wieder in die Basen übergeführt werden. Von den letzteren lassen sich durch Umsetzung mit organischen Säuren, insbesondere solche, die zur Bildung von therapeutisch verwendbaren Salzen geeignet sind, Salze gewinnen.

Die Verbindungen der allgemeinen Formel I können hergestellt werden, indem man eine Verbindung der allgemeinen Formel 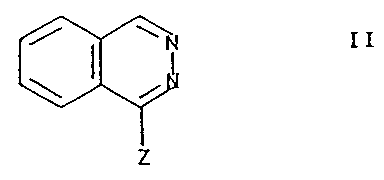 mit einer Verbindung der allgemeinen Formel

R-Y III

umsetzt, wobei R die angegebenen Bedeutungen hat und Z ein Halogenatom ist, falls Y eine Hydroxygruppe, eine C₁-C₆-Alkylsulfonylgruppe oder eine Arylsulfonyl- oxygruppe oder eine Mercaptogruppe bedeutet, oder Z eine Hydroxygruppe oder eine Mercaptogruppe ist, falls Y Halogen bedeutet und gegebenenfalls die erhaltenen Verbindungen alkyliert oder acyliert und/oder in die Säuresalze überführt.

Die Erfindung betrifft auch die Verwendung von Verbindungen der Formel I und zusätzlich die Verbindungen 1-Methoxy-phthalazin, 1-Ethoxy-phthalazin und 1-Phenoxy-phthalazin zur Herstellung von Arzneimitteln.

Das Verfahren zur Herstellung von Verbindungen der Formel II und III wird in einem Lösungs- oder Dispergiermittel bei Temperaturen zwischen 20 und 200 ^{o}C, vorzugsweise 30 und 150 ^{o}C, insbesondere 40 und 80 ^{o}C, durchgeführt. Als Lösungs- beziehungsweise Dispergiermittel kommen zum Beispiel in Frage: niedere aliphatische Alkohole (1-6 C-Atome wie Propanol, Isopropanol, Butanol), niedere aliphatische Ether (Diethylether, Diisopropylether), aromomatische Kohlenwasserstoffe (Benzol, Toluol, Xylol), cyclische Ether (Dioxan, Tetrahydrofuran), Ester von niederen aliphatischen Carbonsäuren mit niederen aliphatischen Alkoholen, Amide und N-alkylsubstituierte Amide von aliphatischen C₁-C₄-Carbonsäuren (Dimethylformamid, Dimethylacetamid), C₁-C₆-Dialkylsulfone (Dimethylsulfoxid) sowie weitere aprotische Mittel wie N-Methylpyrrolidon, Tetramethylharnstoff, Hexamethylphosphorsäuretriamid, Acetonitril. Die einzelnen Alkylreste der oben angegebenen Lösungsmittel enthalten beispielsweise 1-6, insbesondere 1-4 Kohlenstoffatome.
Das Verfahren wird zweckmäßig in Gegenwart von Kondensationsmitteln durchgeführt. Als derartige Kondensationsmittel kommen zum Beispiel in Frage, anorganische Kondensationsmittel wie Alkali- oder Erdalkalihydroxide, Alkalihydride, Alkaliamide, Alkali- oder Erdalkalicarbonate oder organische Basen wie Pyridin, tertiäre Amine, Piperidin, Alkalialkholate, Alkaliacetate oder auch Triethylphosphat. Bei den Alkalimetallen handelt es sich insbesondere um Natrium oder Kalium. Es kann auch unter Phasen-Transfer-Bedingungen ( das heißt unter Zusatz eines oder mehrerer langkettiger Amine wie einem Benzyltributyl-ammonium-halogenid, einem Tetrabutyl-ammonium-halogenid oder Benzyltriphenyl-phosphoniumchlorid gearbeitet werden.

Im allgemeinen stellt man aus der Ausgangskomponente, die die Hydroxy- beziehungsweise Mercaptogruppe enthält, zuerst unter Verwendung einer wie oben angegebenen Alkaliverbindung das entsprechende Salz her und setzt dieses dann anschließend mit der zweiten Reaktionskomponente II um.

Falls Y der Formel III eine C₁-C₆-Alkyl-sulfonyloxygruppe ist, handelt es sich vorzugsweise um eine solche mit 1-4 C-Atomen im Alkylteil (beispielsweise die Methylsulfonyloxygruppe). Falls Y der Formel III eine Arylsulfonyloxygruppe ist, handelt es sich bei dem Arylrest vorzugsweise um einen Phenyl- oder Naphthylrest, wobei diese gegebenenfalls durch C₁-C₄-Alkylreste (insbesondere Methylreste) substituiert sein können (zum Beispiel p-Toluolsulfonyloxygruppe).

Herstellung von Ausgangsstoffen der Formel II, worin Z = SH ist: Solche Verbindungen können beispielsweise aus Verbindungen der Formel II, worin Z ein Halogenatom (Fluor, Chlor, Brom, Jod) ist, durch Umsetzen mit Natrium- oder Kaliummercaptid in Alkoholen (Methanol, Ethanol, Propylenglykol) bei Temperaturen zwischen 20 und 150° C oder auch in wässrigem Medium bei 100 - 150° C oder durch Umsetzung mit Thioharnstoff in niederen Alkoholen (Ethanol, Isopropanol) bei Temperaturen zwischen 20 und 100 ° C und anschließender alkalischer Zersetzung (beispielsweise mit wässrigem Natriumcarbonat auf dem Dampfbad) erhalten werden.

Eine weitere Möglichkeit ist das Erhitzen von Verbindungen der Formel II, worin Z eine Hydroxygruppe ist, mit Phosphorpentasulfid auf Temperaturen zwischen 50 und 200 ° C, zum Beispiel 60 - 160 ° C. Diese Umsetzungen können analog den Verfahren erfolgen, die beispielsweise in Erwin Klingenberg, Pyridine and its Derivatives, Part IV (1964), Seiten 348 - 351 oder der DE-OS 2 230 392, Seite 9 angegeben sind.

Ausgangsstoffe der Formel III, worin Y die Hydroxyruppe ist, können aus solchen Verbindungen der Formel III, worin R Halogen ist, durch alkalische Hydrolyse in an sich bekannter Weise, wie zum Beispiel in C. Ferri, Reaktionen der organischen Synthese, 1978, Seite 200, oder in Houben-Weyl, Methoden der organischen Chemie, Bd. VI/1a, Teil 1, S. 180-191, beschrieben ist, erhalten werden.

Aus Verbindungen der Formel III, worin Y die Hydroxygruppe ist, können solche Ausgangsstoffe der Formel III erhalten werden, worin Y ein Halogenatom ist und zwar beispielsweise durch Umsetzung mit Thionylhalogeniden (Chloriden, Bromiden, Jodiden) oder Sulfonsäurechloriden in Halogenkohlenwasserstoffen (Chloroform) oder aromatischen Kohlenwasserstoffen (Benzol) oder in Pyridin bei Temperaturen zwischen 20 und 150 ° C (vorzugsweise Siedetemperatur des verwendeten Lösungsmittels). Ausgangsstoffe der Formel III, worin Y eine Alkylsulfonyloxygruppe oder eine Arylsulfonyloxygruppe ist, können zum Beispiel aus den entsprecheden Hydroxyverbindungen (Y = OH) durch Umsetzung mit C₁-C₆-Alkyl-sulfonsäurechloriden oder den entsprechenden Arylsulfonsäurechloriden in hierfür üblichen inerten Lösungsmitteln (Benzol, Toluol, Xylol, Chloroform, Methylenchlorid, Dioxan) bei Temperaturen zwischen 20 - 150 ° C erhalten werden. Zweckmäßig wird hierbei in Gegenwart einer säurebindenden Substanz (zum Beispiel tertiäre Amine wie Triethylamin) gearbeitet.

Aus den Halogeniden der Formel III (Y = Halogen) können beispielsweise durch Umsetzung mit Alkalisulfiden Ausgangsstoffe der Formel III erhalten werden, worin Y die Mercaptogruppe ist. Diese Umsetzungen können analog C. Ferri, Reaktionen der organischen Synthese 1978, Seiten 205 - 209 oder analog der DE-OS 2 230 392 zum Beispiel Seite 9 erfolgen.

### Alkylierung und Acylierung

Es handelt sich hierbei um die Acylierung oder Alkylierung von Aminogruppen (zum Beispiel wenn die Reste R₁, R₂ und/oder R₃ Amino-, oder Monoalkylamino-beziehungsweise Dialkylaminogruppen darstellen).
Die Alkylierung erfolgt beispielsweise durch Umsetzung mit Verbindungen der Formel R'Hal, ArSO₂OR' und SO₂(OR'₃)₂, wobei Hal ein Halogenatom (insbesondere Chlor, Brom oder Jod) und Ar ein aromatischer Rest (zum Beispiel ein gegebenenfalls durch einen oder mehrere niedere Alkylreste substituierter Phenyl- oder Naphthylrest) und R' eine C₁-C₆-Alkylgruppe ist. Beispiele sind p-Toluolsulfonsäure-C₁C₆-alkylester, C₁-C₆-Dialkylsulfate, C₁-C₆-Alkylhalogenide.
Die Alkylierungs- und Acylierungsreaktion wird gegebenenfalls unter Zusatz von üblichen säurebindenden Mitteln wie Alkalihydroxiden, Alkalicarbonaten, Alkalihydrogencarbonaten, Erdalkalicarbonaten, Alkaliacetaten, tertiären Aminen (zum Beispiel Trialkylamin wie Triethylamin), Pyridin oder auch Alkalihydriden bei Temperaturen zwischen 0 und 200° C, vorzugsweise 40 und 140°C in inerten Lösungsmitteln oder Suspensionsmitteln durchgeführt. Als Lösungs- oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid; aliphatische Ether wie zum Beispiel Butylether; cyclische Ether wie zum Beispiel Tetrahydrofuran, Dioxan; Sulfoxyde wie zum Beispiel Dimethylsulfoxyd; tertiäre Säureamide wie zum Beispiel Dimethylformamid, N-Methylpyrrolidon, Hexamethylphosphorsäuretriamid; alipathische Alkohole wie Methanol, Ethanol, Isopropanol, Amylalkohol, tertiäres Butanol, cycloaliphatische Kohlenwasserstoffe wie Cyclohexan und ähnliche. Auch wässrige Mischungen der genannten Lösungsmittel können verwendet werden. Häufig arbeitet man bei der Rückflußtemperatur der verwendeten Lösungs-beziehungsweise Dispergiermittel. Häufig werden die Alkylierungsreaktionskomponenten im Überschuß eingesetzt. Die Alkylierung kann auch in Gegenwart von Tetraalkylammoniumsalzen (insbesondere der Halogenide) in Kombination mit Alkalihydroxiden bei Temperaturen zwischen 0 - 100 °C, vorzugsweise 20 - 80 °C, in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-Methyl-Pyrrolidon, Hexamethylphosphorsäuretriamid) Dimethylsulfoxid, Acetonitril, Dimethoxyethan, Aceton, Tetrahydrofuran.

Bei der Acylierung wird zum Beispiel in Verbindungen der Formel I, die Aminogruppen oder NH-Gruppen enthalten, eine C₂-C₆-Alkanoylgruppe eingeführt.

Man verfährt hierbei in an sich bekanner Weise vorzugsweise unter Verwendung von Carb-C₁-C₆-alkoxyhalogeniden (oder der entsprechenden Anhydride) oder unter Verwendung von C₂-C₆-Alkanoylhalogeniden (beziehungsweise entsprechender Anhydride). Die Reaktionstemperaturen liegen vorzugsweise zwischen 30 und 120° C.

Gegebenenfalls kann man bei der Alkylierung und der Acylierung auch so vorgehen, daß man zuerst von der zu alkylierenden beziehungsweise acylierenden Verbindung eine Alkaliverbindung (Natrium-, Kalium- oder auch Lithiumsalz zum Beispiel) herstellt, indem man sie in einem inerten Lösungsmittel wie Dioxan, Dimethylformamid, Benzol oder Toluol mit einem Alkalimetall, Alkalihydrid oder Alkaliamiden (insbesondere Natrium oder Natriumverbindungen) oder Butyllithium bei Temperaturen zwischen 0 und 150° C umsetzt und dann das alkylierende oder acylierende Agens zufügt.

Anstelle der angeführten Alkylierungs- und Acylierungsmittel können auch andere in der Chemie gebräuchliche chemich-äquivalente Mittel verwendet werden (siehe zum Beispiel auch L.F. und Mary Fiser "Reagents for Organic Synthesis", John Wiley and Sons, Inc., New York, 1967, Vol 1, Seiten 1303-4 und Vol. 2, Seite 471.

Falls in Verbindungen der Formel I der Rest R beispielsweise eine Carb-C₁-C₆-Alkoxygruppe oder eine C₂-C₆-Alkanoylgruppe enthält, können diese Gruppen solvolytisch abgespalten werden. Diese Abspaltung erfolgt in bekannter Weise beispielsweise durch Verseifung mit Säuren, (Mineralsäuren wie Salzsäure, Schwefelsäure, insbesondere konzentrierten Halogenwasserstoffsäuren wie HBr/Eisessig) oder mittels basischer Substanzen (Pottasche, Soda, wässrige Alkalilösungen, alkoholische Alkalilösungen, wässriges NH₃) bei Temperaturen zwischen 10 und 150°C, insbesondere 20 bis 100 °C.

Diejenigen Verbindungen der Formel I, die asymmetrische Kohlenstoffatome enthalten und in der Regel als Razemate anfallen, können in an sich bekannter Weise beispielsweise mit Hilfe einer optisch akiven Säure in die optisch aktiven Isomeren gespalten werden. Es ist aber auch möglich, von vornherein eine optisch aktive Ausgangssubstanz einzusetzen, wobei dann als Endprodukt eine entsprechende optisch aktive beziehungsweise diastereomere Form erhalten wird.

Die vorliegende Erfindung umfaßt also auch die D- und L-Formen sowie auch die DL-Mischungen für den Fall, daß in der Verbindung der Formel I ein asymmetrisches C-Atom vorkommt und für den Fall von zwei und mehr asymmetrischen C-Atomen ebenso die entsprechenden diastereomeren Formen.

Die erfindungsgemäßen Verbindungen sind zur Herstellung pharmazeutischer Zusammensetzungen geeignet. Die pharmazeutischen Zusammensetzungen beziehungsweise Medikamente können eine oder mehrere der erfindungsgemäßen Verbindungen enthalten. Zur Herstellung der pharmazeutischen Zubereitungen können die üblichen pharmazeutischen Träger- und Hilfsstoffe verwendet werden. Die Arzneimittel können zum Beispiel enteral, parenteral (zum Beispiel intravenös, intramuskulär, subkutan) oder oral angewendet werden. Beispielsweise kann die Verabreichung von Form von Tabletten, Kapseln, Pillen, Dragees, Zäpfchen oder Pflastern erfolgen. Als Liquida kommen zum Beispiel in Frage: Ölige oder wässrige Lösungen oder Suspensionen (zum Beispiel in Sesam- oder Olivenöl), Emulsionen, injizierbare wässrige oder ölige Lösungen oder Suspensionen. Weiterhin können beispielsweise Trockenampullen, welche als Wirkstoff die erfindungsgemäße Verbindung I enthalten, hergestellt werden, wobei vor Gebrauch der Inhalt solcher Trockenampullen zum Beispiel in Wasser, physiologischer Kochsalzlösung und Dimethylsulfoxid aufgelöst wird.

Die erfindungsgemäßen Verbindungen zeigen beispielsweise im Essigsäure-Writhing Test, im Randall-Selitto Schmerztest sowie im Hefefieber Test eine gute analgetische, antiphlogistische und antipyretische Wirkung. Beispielsweise wird im Essigsäure-Writhing Test bei einer Dosis von 5,6 mg/kg Körpergewicht Maus eine 50%ige Hemmung des Writhingsyndroms (charakteristisches Strecken der Tiere als Schmerzreaktion) erzielt.

Die niedrigste, bereits wirksame Dosis im Essigsäure-Writhing Test ist beispielsweise 3 mg/kg oral.

Als allgemeiner Dosisbereich für die Wirkung (Tierversuch wie oben) kommt beispielsweise in Frage:
5 - 30 mg/kg oral beziehungsweise
3 - 20 mg/kg intravenös.

Die Wirkungsrichtung der erfindungsgemäßen Verbindungen ist mit der Wirkung des bekannten Arzneimittelwirkstoffs Paracetamol, oder Acetylsalicylsäure vergleichbar, jedoch bestehen hierzu insbesondere folgende Unterschiede: stärker und länger anhaltende Wirkung, fehlende gastro-intestinale Nebenwirkungen. Indikationen für die die erfindungsgemäßen Verbindungen in Betracht kommen können: Schmerz, Fieber, Epilepsie.

Die pharmazeutischen Zubereitungen enthalten im allgemeinen zwischen 25 bis 500 vorzugsweise 100 bis 250 mg der erfindungsgemäßen aktiven Komponente(n).

Die Verarbeitung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 100 und 250 mg oder Lösungen, die zwischen 1 bis 10 Gewichtsprozent an aktiver Substanz enthalten.

Die Einzeldosis der erfindungsgemäßen aktiven Komponenten kann beispielsweise liegen
a) bei oralen Arzneiformen zwischen 50 und 400 mg, vorzugsweise 100 - 250 mg
b) bei parenteralen Arzneiformen (zum Beispiel intravenös, intramuskulär) zwischen 25 und 250 mg vorzugsweise 50 - 125 mg
c) bei Arzneiformen zur Inhalation (Lösungen oder Aerosole) zwischen 1 und 10 % vorzugsweise 2 - 5 %
d) bei Arzneiformen zur rektalen oder vaginalen Applikation zwischen 50 und 500 mg vorzugsweise 125 - 500 mg
e) bei Arzneiformen zur lokalen Applikation auf die Haut und Schleimhäute (zum Beispiel in Form von Lösungen, Lotionen, Emulsionen, Salben und so weiter) zwischen 1 und 10 % vorzugsweise 2 - 5 %
   -(Die Dosen sind jeweils bezogen auf die freie Base)-

Beispielsweise können 3mal täglich 1 bis 2 Tabletten mit einem Gehalt von 50 bis 250 mg wirksamer Substanz oder zum Beispiel bei intravenöser Injektion 1 bis 2 mal täglich eine Ampulle von 3 bis 5 ml Inhalt mit 25 bis 250 mg Substanz empfohlen werden. Bei oraler Verabreichung ist die minimale tägliche Dosis beispielsweise 150 mg; die maximale tägliche Dosis bei oraler Verabreichung soll nicht über 1500 mg liegen.

Die akute Toxizität der erfindungsgemäßen Verbindungen an der Maus (ausgedrückt durch die LD 50 mg/kg-Methode nach Miller und Tainter: Proc. Soc. Exper. Bio. a. Med. 57 (1944) 261) liegt beispielsweise bei oraler Applikation oberhalb 600 mg/kg per os.

Die Arzneimittel können in der Humanmedizin, der Veterinärmedizin sowie in der Landwirtschaft allein oder im Gemisch mit anderen pharmakologisch aktiven Stoffen verwendet werden.

### Die folgenden Beispiele dienen der Erläuterung der Erfindung

### Beispiele:

### Allgemeine Vorschrift zur Herstellung der Beispiele 1 - 15 gemäß der Tabelle 1:

0,30 mol Verbindung III werden in 100 ml Dimethylacetamid (abgekürzt DMA) gelöst und unter Kühlung (Eiswasser) zu einer Suspension von 0,33 mol Natriumhydrid in 70 ml DMA zugetropft. Die Apparatur wird vorher mit Argon gespült. Nach Beendigung der exothermen Reaktion (1,5 - 2 Stunden) wird hierzu eine Lösung von 0,30 mol 1-Chlorphthalazin (95 %ig) in 230 ml DMA bei 20 bis 40° während zwei Stunden zugetropft. Anschließend wird die Reaktionsmischung noch etwa 5 Stunden bei einer Badtemperatur von 40 - 80 °C gerührt.

Nach Beendigung der Reaktion werden unter Eiskühlung ungefähr 50 ml Wasser zu der Reaktionsmischung zugetropft, und anschließend am Rotationsverdampfer im Wasserstrahlvakuum bei Raumtemperatur bis auf ein Volumen von etwa 150 ml eingeengt. Die so konzentrierte Reaktionsmischung wird dann unter kräftigem Rühren in 1 Liter Eiswasser gegossen. Das ausgefallene Reaktionsprodukt wird abgesaugt und mit Wasser, gegebenenfalls anschließend mit Petroläther gewaschen.
Das so erhaltene Rohprodukt wird gegebenenfalls unter Zugabe von Aktivkohle und/oder Kieselgur in üblicher Weise umkristallisiert.

Die hergestellten Verbindungen sind in Tabelle 1 aufgeführt. Bei den Verbindungen gemäß den Beispielen 1 bis 14 ist X jeweils Sauerstoff. Bei Beispiel 15 ist X = Schwefel.

**Tabelle 1**

| Beispiele 1 - 15 | | | |
|---|---|---|---|
| Beispiel Nr. | R | Schmelzpunkt | umkristallisiert Ausbeute aus |
| 1 | C₆H₅- | 105-106°C | 2-Propanol |
| 2 | 4-CH₃0-C₆H₄- | 149-151°C | Ethanol |
| 3 | 4-NH₂-C₆H₄- | 195-196°C | Ethanol |
| 4 | 4-CH₃-C0-NH-C₆H₄ | 213°C | Ethanol |
| 5 | 3-CH₃-C0-NH-C₆H₄ | 232-234°C | Ethanol |
| 6 | C₂H₅- | 57°C | Petrolether |
| 7 | 2,3,4-(CH₃0)₃-C₆H₂ | 183°C | Ethanol |
| 8 | C₆H₅-CH₂- | braune Flüssigkeit | gereinigt über Säulenchromatographie |
| 9 | 4-CH₃0-C0-C₆H₄ | 168-169° | Methanol |
| 10 | 4-(NH₂-CH₂-C0-NH)-C₆H₄ | >248°C (Zers.) Hydrochlorid | Salzbildung in MeOH mit HCl/2 Propanol |
| 11 | 2-Pyridyl-CH₂-(2-Picolyl-) | 99-102°C (Base) 114-115°C (Maleat) | DMA/Wasser¹⁾ Salzbildung des Maleats in Aceton mit Maleinsäure /Aceton |
| 12 | 4-Pyridyl-CH₂-(4-Picolyl-) | 134 -135°C | DMA/Wasser¹⁾ |
| 13 | 1-Aza-bicyclo 2,2,2 -octan-3-yl(3-Chinuclidyl) | 230-231°C Hydrochlorid | Salzbildung in 2-Propanol mit HCl/2-Propanol |
| 14 | 4-F-C₆H₄ | 141-144°C | 2-Propanol |
| 15 | C₆H₅(X=S) | 129-132°C | DMA/Wasser¹⁾ |

| | | | |
|---|---|---|---|
| ¹⁾ ausgefällt durch Zugabe von Wasser zur Reaktions-Lösung | | | |

## Patentansprüche

1. Verbindungen der allgemeinen Formel 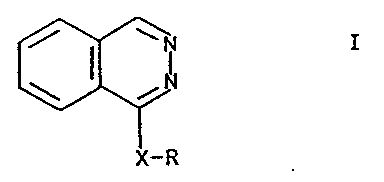 worin X Sauerstoff bedeutet und der Rest R einen Chinuclidylrest,einen Phenylrest, einen Pyridylrest, einen durch die Reste R₁, R₂ und/oder R₃ substituierten Phenyl- oder Pyridylrest, einen durch Pyridyl oder Alkylpyridyl substituierten C₁-C₆-Alkylrest oder einen durch Phenyl substituierten C₁-C₆-Alkylrest darstellt, wobei jener Phenylrest auch durch die Reste R₁, R₂ und/oder R₃ substituiert sein kann und die Reste R₁, R₂ und R₃ gleich oder verschieden sind und Wasserstoff, Halogen, Trihalogenmethyl, C₁-C₆-Alkyl, C₁-C₆-Alkoxy, Carboxy,Carb-C₁-C₆-alkoxy, Amino, C₁-C₆-Alkylamino, C₁-C₆-Dialkylamino, C₁-C₆-Trialkylamino, C₂-C₆-Alkanoylamino oder C₂-C₆-Alkanoylamino, welches im Alkylteil noch eine Aminogruppe enthält, bedeuten und deren physiologisch verträgliche Säureadditionssalze, ausgenommen 1-Phenoxy-phthalazin.

2. Verfahren zur Herstellung von Verbindungen der allgemeinen Formel 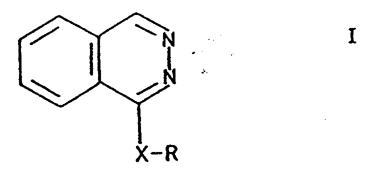 worin X Sauerstoff bedeutet und der Rest R einen Chinuclidylrest, einen Phenylrest, einen Pyridylrest, einen durch die Reste R₁, R₂ und/oder R₃ substituierten Phenyl- oder Pyridylrest, einen durch Pyridyl oder Alkylpyridyl substituierten C₁-C₆-Alkylrest oder einen durch Phenyl substituierten C₁-C₆-Alkylrest darstellt, wobei jener Phenylrest auch durch die Reste R₁, R₂ und/oder R₃ substituiert sein kann und die Reste R₁, R₂ und R₃ gleich oder verschieden sind und Wasserstoff, Halogen, Trihalogenmethyl, C₁-C₆-Alkyl, C₁-C₆-Alkoxy, Carboxy, Carb-C₁-C₆-alkoxy, Amino, C₁-C₆-Alkylamino, C₁-C₆-Dialkylamino, C₁-C₆-Trialkylamino, C₂-C₆-Alkanoylamino oder C₂-C₆-Alkanoylamino, welches im Alkylteil noch eine Aminogruppe enthält, bedeuten und deren physiologisch verträgliche Säureadditionssalze , ausgenommen 1-Phenoxyphthalazin dadurch gekennzeichnet, daß man eine Verbindung der allgemeinen Formel 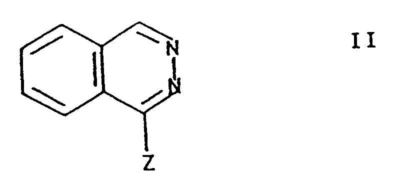 mit einer Verbindung der allgemeinen Formel
R-Y III
umsetzt, wobei R die angegebenen Bedeutungen hat und Z ein Halogenatom ist, falls Y eine Hydroxygruppe, eine C₁-C₆-Alkylsulfonylgruppe oder eine Arylsulfonyloxygruppe oder eine Mercaptogruppe bedeutet, oder Z eine Hydroxygruppe oder eine Mercaptogruppe ist, falls Y Halogen bedeutet und gegebenenfalls die erhaltenen Verbindungen alkyliert oder acyliert und/oder in die Säuresalze überführt.

3. Arzneimittel enthaltend als Wirkstoff mindestens eine Verbindung der Formel 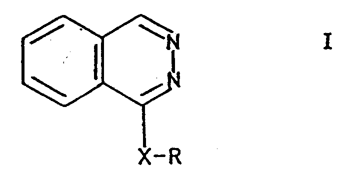 worin X Sauerstoff bedeutet und der Rest R einen Chinuclidylrest, einen Phenylrest, einen Pyridylrest, einen durch die Reste R₁, R₂ und/oder R₃ substituierten Phenyl-oder Pyridylrest, einen durch Pyridyl oder Alkylpyridyl substituierten C₁-C₆-Alkylrest oder einen durch Phenyl substituierten C₁-C₆-Alkylrest darstellt, wobei jener Phenylrest auch durch die Reste R₁, R₂ und/oder R₃ substituiert sein kann und die Reste R₁, R₂ und R₃ gleich oder verschieden sind und Wasserstoff, Halogen, Trihalogenmethyl, C₁-C₆-Alkyl, C₁-C₆-Alkoxy, Carboxy, Carb-C₁-C₆-alkoxy, Amino, C₁-C₆-Alkylamino, C₁-C₆-Dialkylamino, C₁-C₆-Trialkylamino, C₂-C₆-Alkanoylamino oder C₂-C₆-Alkanoylamino, welches im Alkylteil noch eine Aminogruppe enthält, bedeuten und deren physiologish verträgliche Säureadditionssalze.

4. Verfahren zur Herstellung eines Arzneimittels, dadurch gekennzeichnet, daß mindestens ein Wirkstoff gemäß Anspruch 1 unter Einschluß der Verbindungen 1-Methoxy-phthalazin, 1-Ethoxy-phthalazin und 1-Phenoxy-phthalazin mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

5. Verwendung von Verbindungen gemäß Anspruch 1 und zusätzlich die Verbindungen 1-Methoxy-phthalazin, 1-Ethoxy-phthalazin und 1-Phenoxy-phthalazin zur Herstellung von Arzneimitteln.

## Claims

1. Compounds of the general formula 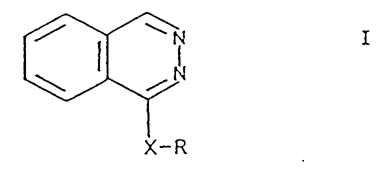 in which X means oxygen and the residue R represents a quinuclidinyl residue, a phenyl residue, a pyridyl residue, a phenyl or pyridyl residue substituted by the residues R₁, R₂ and/or R₃, a C₁-C₆ alkyl residue substituted by pyridyl or alkylpyridyl or a C₁-C₆ alkyl residue substituted by phenyl, wherein each phenyl residue may also be substituted by the residues R₁, R₂ and/or R₃ and the residues R₁, R₂ and R₃ are identical or different and mean hydrogen, halogen, trihalomethyl, C₁-C₆ alkyl, C₁-C₆ alkoxy, carboxy, carb-C₁-C₆-alkoxy, amino, C₁-C₆ alkylamino, C₁-C₆ dialkylamino, C₁-C₆ trialkylamino, C₂-C₆ alkanoylamino or C₂-C₆ alkanoylamino which still contains an amino group in the alkyl portion, and the physiologically compatible acid addition salts thereof, with the exception of 1-phenoxyphthalazine.

2. Process for the production of compounds of the general formula 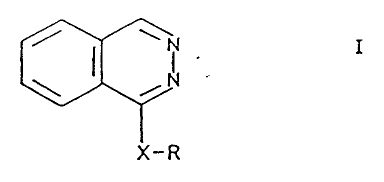 in which X means oxygen and the residue R represents a quinuclidinyl residue, a phenyl residue, a pyridyl residue, a phenyl or pyridyl residue substituted by the residues R₁, R₂ and/or R₃, a C₁-C₆ alkyl residue substituted by pyridyl or alkylpyridyl or a C₁-C₆ alkyl residue substituted by phenyl, wherein each phenyl residue may also be substituted by the residues R₁, R₂ and/or R₃ and the residues R₁, R₂ and R₃ are identical or different and mean hydrogen, halogen, trihalomethyl, C₁-C₆ alkyl, C₁-C₆ alkoxy, carboxy, carb-C₁-C₆-alkoxy, amino, C₁-C₆ alkylamino, C₁-C₆ dialkylamino, C₁-C₆ trialkylamino, C₂-C₆ alkanoylamino or C₂-C₆ alkanoylamino which still contains an amino group in the alkyl portion, and the physiologically compatible acid addition salts thereof, with the exception of 1-phenoxyphthalazine, characterised in that a compound of the general formula 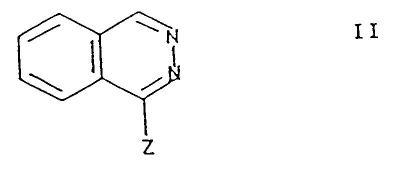 is reacted with a compound of the general formula
R-Y III
wherein R has the stated meaning and Z is a halogen atom, if Y means a hydroxy group, a C₁-C₆ alkylsulphonyl group or an arylsulphonyloxy group or a mercapto group, or Z is a hydroxy group or a mercapto group, if Y means halogen, and the resultant compounds are optionally alkylated or acylated and/or converted into the acid salts.

3. Pharmaceutical containing as active ingredient at least one compound of the formula 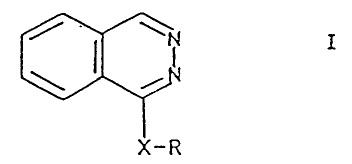 in which X means oxygen and the residue R represents a quinuclidinyl residue, a phenyl residue, a pyridyl residue, a phenyl or pyridyl residue substituted by the residues R₁, R₂ and/or R₃, a C₁-C₆ alkyl residue substituted by pyridyl or alkylpyridyl or a C₁-C₆ alkyl residue substituted by phenyl, wherein each phenyl residue may also be substituted by the residues R₁, R₂ and/or R₃ and the residues R₁, R₂ and R₃ are identical or different and mean hydrogen, halogen, trihalomethyl, C₁-C₆ alkyl, C₁-C₆ alkoxy, carboxy, carb-C₁-C₆-alkoxy, amino, C₁-C₆ alkylamino, C₁-C₆ dialkylamino, C₁-C₆ trialkylamino, C₂-C₆ alkanoylamino or C₂-C₆ alkanoylamino which still contains an amino group in the alkyl portion, and the physiologically compatible acid addition salts thereof.

4. Process for the production of a pharmaceutical, characterised in that at least one active ingredient according to claim 1, including the compounds 1-methoxyphthalazine, 1-ethoxyphthalazine and 1-phenoxyphthalazine are converted with conventional pharmaceutical excipients and/or diluents or other auxiliary substances into pharmaceutical preparations or into a therapeutically usable form.

5. Use of compounds according to claim 1 and additionally the compounds 1-methoxyphthalazine, 1-ethoxyphthalazine and 1-phenoxyphthalazine for the production of pharmaceuticals.

## Revendications

1. Composés de formule générale : 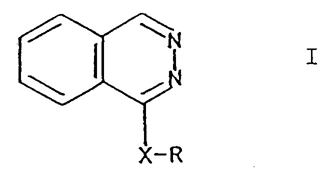 dans laquelle X signifie de l'oxygène
et le reste R un radical quinuclidyle, un radical phényle, un radical pyridyle, un radical phényle ou pyridyle qui peut être substitué par des restes R₁, R₂ et/ou R₃, un radical alcoyle en C₁-C₆ substitué par un pyridyle ou par un alcoylpyridyle, ou un radical alcoyle en C₁-C₆ substitué par un phényle, dans laquelle chaque radical phényle peut aussi être substitué par les restes R₁, R₂ et/ou R₃ et les restes R₁, R₂ et R₃ sont identiques ou différents et signifient de l'hydrogène, un halogène, un trihalogénométhyle, un alcoyle en C₁-C₆, un alcoxy en C₁ à C₆, un carboxy, un carb-alcoxy en C₁-C₆, un amino, un (alcoyl en C₁-C₆)amino, un (dialcoyl en C₁-C₆)amino, un (trialcoyl en C₁-C₆)amino, un alcanoyl en C₂C₆ amino, ou un alcanoyl en C₂-C₆ amino qui dans la partie alcoyle renferme encore un groupe amino, ainsi que leurs sels d'addition avec un acide physiologiquement compatible, à l'exclusion de la 1-phénoxyphthalazine.

2. Procédé de fabrication des composés de formule générale I, 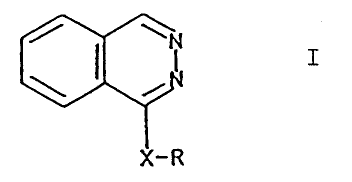 dans laquelle X signifie de l'oxygène
et le reste R représente un radical quinuclidyl, un radical phényle, un radical pyridyle, un radical phényle ou pyridyle substitué par les restes R₁, R₂ et/ou R₃, un radical alcoyle en C₁-C₆ substitué par un pyridyle ou par un alcoylpyridyle, ou un radical alcoyle en C₁-C₆ substitué par un phényle, dans lesquels chaque radical phényle peut être aussi substitué par les restes R₁, R₂ et/ou R₃ et les restes R₁, R₂ et R₃ sont identiques ou différents et signifient de l'hydrogène, un halogène, un trihalogénométhyle, un alcoyle en C₁-C₆, un alcoxy en C₁-C₆, un carboxy, un carb-alcoxy en C₁-C₆, un amino, un (alcoyl en C₁-C₆) amino, un di(alcoyl en C₁-C₆))amino, un tri(alcoyl en C₁-C₆)amino, un alcanoyl en C₂-C₆)amino ou un (alcanoyl en C₂-C₆)amino qui dans la partie alcoyle renferme encore un groupe amino, ainsi que leurs sels d'addition avec un acide physiologiquement compatible
à l'exclusion de la 1-phénoxyphtalazine,
caractérisé en ce que
l'on fait réagir un composé de formule générale : 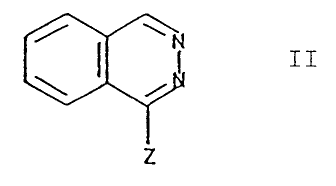 avec un composé de formule générale III,
R-Y III
dans laquelle R a les significations indiquées et Z est un atome d'halogène au cas où Y est un groupe hydroxy, un groupe (alcoyle en C₁-C₆) sulfonyle ou un groupe arylsulfonyloxy [ou un groupe mercapto],
ou Z est un groupe hydroxy [ou mercapto] au cas où Y signifie un halogène
et en ce qu'éventuellement on alcoyle ou acyle les composés obtenus et/ou les convertit en sels avec un acide.

3. Médicament contenant comme principe actif au moins un composé de formule : 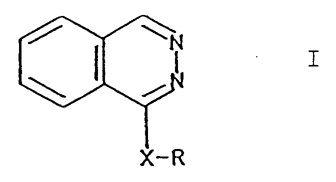 et le reste R un radical quinuclidyle, un radical phényle, un radical pyridyle, un radical phényle ou pyridyle substitué par les restes R₁, R₂ et/ou R₃, un radical alcoyle en C₁-C₆ substitué par un pyridyle ou par un alcoylpyridyle, ou un radical alcoyle en C₁-C₆ substitué par un phényle, dans laquelle chaque radical phényle peut être aussi substitué par les restes R₁, R₂ et/ou R₃ et les restes R₁, R₂ et R₃ sont identiques ou différents et signifient de l'hydrogène, un halogène, un trihalogénométhyle, un alcoyle en C₁-C₆, un alcoxy en C₁-C₆, un carboxy, un carb-alcoxy en C₁-C₆, un amino, un (alcoyl en C₁-C₆) amino, un di(alcoyl en C₁-C₆))amino, un tri(alcoyl en C₁-C₆)amino, un (alcanoyl en C₂-C₆)amino ou un (alcanoyl en C₂-C₆)amino qui dans la partie alcoyle renferme encore un groupe amino,
- et leurs sels d'addition avec un acide physiologiquement compatible.

4. Procédé d'obtention d'un médicament,
caractérisé en ce que
l'on transforme au moins un principe actif conformément à la revendication 1 y compris les composés 1-méthoxyphthalazine, 1-éthoxyphthalazine, et 1-phénoxyphthalazine, avec des substances support et/ou des agents diluants, pharmaceutiques usuels, ou des substances auxiliaires quelconques, en préparations pharmaceutiques et on met en une forme thérapeutiquement utilisable.

5. Utilisation des composés conformément à la revendication 1 et en supplément les composés 1-méthoxyphthalazine, 1-éthoxyphthalazine, et 1-phénoxyphthalazie en vue de la fabrication de médicaments.
